(19) **Europäisches Patentamt / European Patent Office / Office européen des brevets**

(11) **EP 3 355 056 A1**

(12) **EUROPEAN PATENT APPLICATION**

(43) Date of publication:
**01.08.2018 Bulletin 2018/31**

(51) Int Cl.:
***G01N 33/28*** (2006.01)

(21) Application number: **17153885.3**

(22) Date of filing: **31.01.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Auramarine Oy**
**20660 Littoinen (FI)**

(72) Inventors:
• **Salo, Jouko**
**20100 Turku (FI)**
• **Lehto, Tony**
**21530 Paimio (FI)**

(74) Representative: **Suominen, Kaisa Liisa**
**Moosedog Oy**
**Rykmentintie 2B**
**20810 Turku (FI)**

(54) **SYSTEM AND METHOD FOR MEASURING SULPHUR CONTENT IN PETROCHEMICAL PRODUCTS**

(57)    Disclosed is a system (100) for a continuous measurement of sulphur content of a combustion fuel. The system comprises a burning chamber (102). The burning chamber comprises an air inlet (104). Further, the burning chamber comprises an inlet (106) for a first reference fuel. Furthermore, the burning chamber comprises an inlet (108) for the combustion fuel. Additionally, the burning chamber comprises a means (110) to ignite the combustion fuel. Further, the burning chamber comprises an outlet (112) for exhaust gas from the burning. Furthermore, the outlet comprises a means for preventing condensation. Additionally, the system comprises a means (114) for measuring a sulphur dioxide/carbon dioxide ratio of the exhaust gas. Further, the system comprises a control system (116) configured to compare the measured sulphur dioxide/carbon dioxide ratios.

**FIG. 1**

EP 3 355 056 A1

**Description**

TECHNICAL FIELD

The present disclosure relates generally to instruments and apparatus for measuring; and more specifically, to systems and methods for measuring sulphur content in petrochemical products.

BACKGROUND

Petrochemical products are extensively used in a wide variety of industrial and commercial applications. For example, the transportation industry is the largest worldwide consumer of crude oil and its derivatives.

Petrochemical products contain several impurities, such as for example water, sulphur compounds, oxygen, nitrogen, carbon dioxide and traces of metals. Accordingly, machinery and processes are used to purify petrochemical products. However, the cost of purifying petrochemical products is often prohibitive in several applications. For instance, in the transport industry, it is common for cargo ships to operate on fuel oils containing impurities such as sulphur. While use of less refined fuel oils saves cost, it results is a negative environmental impact.

For example, when fuel oil containing sulphur is burned, sulphur dioxide is produced as a combustion by-product. Sulphur dioxide then reacts with water in the atmosphere producing sulphuric acids, which are environmentally harmful. Further, burning of such fuel oil also produces airborne sulfate particulates that are detrimental when inhaled by living beings.

Accordingly, regulatory bodies impose limits on sulphur content in fuels used in vehicles and cargo ships. For example, the International Maritime Organisation (IMO) currently restricts the sulphur content in heavy fuel oils used on all oceans to 3.5 %. Further, certain regions called as Sulphur Emission Control Areas (SECA) have been established with even further reduced sulphur limit. For example, since January 2015, the sulphur limit has been set to 0.1 % in SECA designated zones, such as, the Baltic Sea, the North Sea, North American Emission Control Area (ECA) and the US Caribbean ECA. These regulations have become more stringent in the recent years and non-compliance with the regulations has the potential for large penalties for fuel users.

Currently, fuel consumers rely on certificates provided by fuel suppliers and/or third parties in order to ensure compliance with regulations regarding emission control. Further, fuel consumers may also conduct X-ray fluorescence analysis of a sample of a fuel in order to be certain that the fuel meets the limits on impurities, such as sulphur content. However, such existing techniques suffer from several limitations.

Firstly, relying on certificates is risk prone. Secondly, obtaining such certificates on a regular basis from third parties is both time consuming and expensive. Thirdly, installing and operating X-ray fluorescence analysis equipment at the place of fuel consumption, such as cargo ships is costly. Fourthly, there are several applications where fuels with different compositions are mixed and used. Further, the composition of such a fuel mixture may vary in time as fuel batches might not completely mix in the fuel tanks. Therefore, one problem faced by fuel consumers is to measure sulphur content in petrochemical products (such as marine fuel) in a substantially continuous manner.

Continuous measurement is important because operators need to be able to adjust fuel handling processes based on the measurement. As an example, a ship sailing into a zone designated as SECA must have heavy fuel oil containing high sulphur purged from the fuel oil system before entering the zone. Currently the ship's operator cannot know for certain that this is the case as there are no methods and/or apparatus to monitor sulphur content on a continuous basis. A similar situation is present when the ship is loading fuel. The operator cannot know the sulphur content of the fuel in real-time but must wait for a certificate given by a third party for a fuel sample.

Therefore, in light of the foregoing discussion, there exists a need to overcome the aforementioned drawbacks by continuously monitoring sulphur content in petrochemical products.

SUMMARY

The present disclosure seeks to provide a system for a continuous measurement of sulphur content of a combustion fuel. Further, the present disclosure seeks to provide a method for a continuous measurement of sulphur content of a combustion fuel. The present disclosure seeks to overcome the problems in prior art, and to provide a cost-effective way of continuously measuring sulphur content of a combustion fuel. One aim is to provide a method that can be fully automated and that is easy to monitor, and which further gives reliable information in an informative manner.

In one aspect, an embodiment of the present disclosure provides a system for a continuous measurement of sulphur content of a combustion fuel. The system comprises a burning chamber. The burning chamber comprises an air inlet, an inlet for a first reference fuel, an inlet for the combustion fuel, means to ignite the combustion fuel and an outlet for exhaust gas from the burning. Furthermore, the outlet comprises means for preventing condensation. Additionally, the system comprises means for measuring a sulphur dioxide/carbon dioxide ratio of the exhaust gas. Further, the system comprises a control system configured to compare the measured sulphur dioxide/carbon dioxide ratios.

In another aspect, an embodiment of the present disclosure provides a vessel comprising a system as described above.

In another aspect, an embodiment of the present disclosure provides a method for a continuous measurement of sulphur content of a combustion fuel. Accordingly, the method comprises burning a first amount of a first reference fuel to produce a first reference exhaust gas and measuring a sulphur dioxide/carbon dioxide ratio of the first reference exhaust gas. Further, the method comprises burning a third amount of the combustion fuel to produce a combustion exhaust gas and measuring a sulphur dioxide/carbon dioxide ratio of the combustion exhaust gas. Furthermore, the method comprises comparing the sulphur dioxide/carbon dioxide ratio of the first reference exhaust gas and the sulphur dioxide/carbon dioxide ratio of the combustion exhaust gas. Additionally, the method comprises instructing an actioning system if the sulphur dioxide/carbon dioxide ratio of the combustion exhaust gas exceeds the sulphur dioxide/carbon dioxide ratio of the first reference exhaust gas.

In another aspect, an embodiment of the present disclosure provides another method for a continuous measurement of sulphur content of a combustion fuel. The method comprises burning a first amount of a first reference fuel to produce a first reference exhaust gas and measuring a sulphur dioxide/carbon dioxide ratio of the first reference exhaust gas. Further, the method comprises burning a second amount of a second reference fuel to produce a second reference exhaust gas and measuring a sulphur dioxide/carbon dioxide ratio of the second reference exhaust gas. Additionally, the method comprises burning a third amount of the combustion fuel to produce a combustion exhaust gas and measuring a sulphur dioxide/carbon dioxide ratio of the combustion exhaust gas. Further, the method comprises using interpolation to convert the sulphur dioxide/carbon dioxide ratio of the combustion exhaust gas to a sulphur content of the combustion fuel. Furthermore, the method comprises instructing an actioning system if the sulphur content of the combustion fuel exceeds a pre-defined limit.

Embodiments of the present disclosure substantially eliminate or at least partially address the aforementioned problems in the prior art, and enable continuous monitoring of sulphur content in petrochemical products. Such monitoring may be performed in a simple and cost effective manner. Accordingly, expensive instruments such as those for performing for example X-ray fluorescence analysis, may be avoided.

Additional aspects, advantages, features and objects of the present disclosure would be made apparent from the drawings and the detailed description of the illustrative embodiments construed in conjunction with the appended claims that follow.

It will be appreciated that features of the present disclosure are susceptible to being combined in various combinations without departing from the scope of the present disclosure as defined by the appended claims.

BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the present disclosure is not limited to specific methods and instrumentalities disclosed herein. Moreover, those in the art will understand that the drawings are not to scale. Wherever possible, like elements have been indicated by identical numbers.

Embodiments of the present disclosure will now be described, by way of example only, with reference to the following diagrams wherein:

FIG. 1    is a schematic illustration of a system for a continuous measurement of sulphur content of a combustion fuel based on a single reference fuel, according to an embodiment.

FIG. 2    is a schematic illustration of a system for a continuous measurement of sulphur content of a combustion fuel based on two reference fuels, according to an embodiment.

FIG. 3    is a schematic illustration of a system for a continuous measurement of sulphur content of a combustion fuel including an actioning means, according to an embodiment.

FIG. 4    is a schematic illustration of a system for a continuous measurement of sulphur content of a combustion fuel including flow control elements, according to an embodiment.

FIG. 5    is a schematic illustration of a system for performing a continuous measurement of sulphur content of a combustion fuel, according to an embodiment.

In the accompanying drawings, an underlined number is employed to represent an item over which the underlined number is positioned or an item to which the underlined number is adjacent. A non-underlined number relates to an item identified by a line linking the non-underlined number to the item. When a number is non-underlined and accompanied by an associated arrow, the non-underlined number is used to identify a general item at which the arrow is pointing.

DETAILED DESCRIPTION OF EMBODIMENTS

The following detailed description illustrates embodiments of the present disclosure and ways in which they can be implemented. Although some modes of carrying out the present disclosure have been disclosed, those skilled in the art would recognize that other embodiments for carrying out or practicing the present disclosure are also possible.

In one aspect, an embodiment of the present disclosure provides a system for a continuous measurement of sulphur content of a combustion fuel. Such a system may be used to measure and control fuel quality while tanking by analysing a sample of the combustion fuel, or when the fuel is being directed to a motor for being burned. Additionally, the system may be used to control fuel mixing (i.e. fuel from two or more tanks) in a combustion engine of a vessel. Accordingly, in an embodiment, the system may be part of the vessel, such as, for example, a cargo ship, a submarine, a land vehicle, an aircraft etc.

In an embodiment, the system may be arranged at one or more locations selected from a combustion fuel tank inlet, a combustion fuel tank outlet and a combustion engine fuel inlet. As a result, for example, when fuel tank of the vessel is being filled at a harbour, the quality of the fuel, specifically its sulphur content, may be continuously monitored. Accordingly, severe penalties of using high sulphur content fuel in SECA designated zones may be avoided.

In order to burn fuel, the system comprises a burning chamber. The burning chamber in general is a substantially insulated chamber configured to carry out combustion of fuel. For example, the burning chamber may be an industrial oil burner, a combustion engine etc.

In order to facilitate combustion, the burning chamber comprises an air inlet. The air inlet is configured for providing a constant air pressure to the burning chamber. For example, an air pump or fan may be used for providing the constant air pressure. Further, in an embodiment, the burning chamber may comprise an electrically controllable valve or pump fluidly coupled with the air inlet. Furthermore, the electrically controllable valve or pump may be configured to control flow of air into the air inlet based on electrical signals. Accordingly, one or more of an amount of air, a pressure of air and a flow of air in the burning chamber may be controlled.

Further, the burning chamber comprises an inlet for a first reference fuel. The first reference fuel may comprise one or more of a fuel with zero sulphur content such as ethanol, and a fuel with known sulphur content, such as DMX grade marine oil. Advantages of using ethanol as the first reference fuel comprise wide availability and clean burning with little residue. The sulphur content in the first reference fuel may be measured accurately to calibrate analysis parameters of the system. Table 1 illustrates commonly used grades of marine oil fuels DMX, DMA, DMZ and DMB.

Table 1

| Parameter | Unit | Limit | DMX | DMA | DMZ | DMB |
|---|---|---|---|---|---|---|
| Viscosity at 40°C | mm$^2$/s | Max | 5.500 | 6.000 | 6.000 | 11.00 |
| Viscosity at 40°C | mm$^2$/s | Min | 1.400 | 2.000 | 3.000 | 2.000 |
| Micro Carbon Residue at 10 % Residue | % m/m | Max | 0.30 | 0.30 | 0.30 | - |
| Density at 15°C | Kg/m$^3$ | Max | - | 890.0 | 890.0 | 900.0 |
| Micro Carbon Residue | % m/m | Max | - | - | - | 0.30 |
| Sulphur | % m/m | Max | 1.00 | 1.50 | 1.50 | 2.00 |
| Water | % V/V | Max | - | - | - | 0.30 |
| Total sediment by hot filtration | % m/m | Max | - | - | - | 0.10 |
| Ash | % m/m | Max | 0.010 | 0.010 | 0.010 | 0.010 |
| Flash point | 0°C | Min | 43.0 | 60.0 | 60.0 | 60.0 |
| Pour point, Summer | 0°C | Max | 0 | 0 | 0 | 6 |
| Pour point, Winter | °C | Max | -6 | -6 | -6 | 0 |
| Cloud point | °C | Max | -16 | - | - | - |
| Calculated Cetane Index | | Min | 45 | 40 | 40 | 35 |

(continued)

| Parameter | Unit | Limit | DMX | DMA | DMZ | DMB |
|---|---|---|---|---|---|---|
| Acid Number | mgKOH/g | Max | 0.5 | 0.5 | 0.5 | 0.5 |
| Oxidation stability | g/m$^3$ | Max | 25 | 25 | 25 | 25 |
| Lubricity, corrected wearscardiameter (wsd 1.4 at 60°C) | um | Max | 520 | 520 | 520 | 520 |
| Hydrogen sulphide | mg/kg | Max | 2.00 | 2.00 | 2.00 | 2.00 |
| Appearance | | | Clear & Bright | | | |

In general, the first reference fuel may be such that complete burning may be accomplished. Further, the chemical composition of the first reference fuel, especially its carbon [C] / hydrogen (H) relationship may be known so that measurement of sulphur content can be linearized.

Further, the system may comprise a container for storing the first reference fuel. Additionally, the system may comprise a flow control element, such as an electrically controlled valve or pump, configured for controlling supply of the first reference fuel from container to the burning chamber. Accordingly, one or more of a quantity, a pressure and a flow of the first reference fuel in the burning chamber may be controlled.

Furthermore, the burning chamber comprises an inlet for the combustion fuel. In general, the combustion fuel may comprise one or more petroleum products based on hydrocarbons. Examples of the combustion fuel may include but are not limited to petrol, diesel, kerosene, fuel oil, crude oil, sour crude oil, sweet crude oil, bunker fuel, furnace fuel oil, marine gas oil, marine diesel oil, intermediate fuel oil, marine fuel oil, heavy fuel oil, light fuel oil, heating oil, jet fuel, Naptha, coconut oil etc.

Further, the system may comprise a container for storing the combustion fuel, or the combustion fuel may be taken from the fuel tank, i.e. the same tank from which it is directed to the motors. Additionally, the system may comprise a flow control element, such as an electrically controlled valve or pump, configured for controlling supply of the combustion fuel from the container to the burning chamber. Accordingly, one or more of a quantity, a pressure and a flow of the combustion fuel in the burning chamber may be controlled.

Additionally, the burning chamber comprises means to ignite the combustion fuel. Accordingly, the burning chamber may comprise a glow cylinder arranged to be heated by for example an ethanol flame. Accordingly, the system may comprise a container for storing ethanol (95 %). Further, an outlet of this container may be coupled to the burning chamber to supply ethanol into the burning chamber. Furthermore, the system may comprise a flow control element, such as an electrically controlled valve or pump, configured for controlling supply of ethanol from the container to the burning chamber. Accordingly, one or more of a quantity, a pressure and a flow of ethanol in the burning chamber may be controlled. The ethanol used for the glow cylinder can naturally be the same ethanol that is used as the first reference fuel.

Alternatively, the glow cylinder may be electrically heated. Further, the burning chamber may comprise a burn pot in which each of the first reference fuel and the combustion fuel is burnt. Accordingly, the glow cylinder is configured to heat each of the first reference fuel and the combustion fuel.

Further, the burning chamber comprises an outlet for exhaust gas from the burning. Furthermore, the outlet comprises means for preventing condensation. In an embodiment, the means for preventing condensation may be selected from heating means and thermal insulation means. Sulphur oxides are prone to dissolve to water. Accordingly, it is important that no cold surfaces are in contact with the exhaust gas to avoid condensing. Therefore, it is advantageous to insulate a pipe carrying the exhaust gas to prevent heat leakage. Further, one or more of the pipe and associated components may comprise heating, such as for example by electricity.

Additionally, the outlet may comprise a flow restrictor orifice configured to restrict flow of the exhaust gas in order to facilitate intake of the exhaust gas into a gas inlet. For example, the flow restrictor orifice may comprise an orifice plate dimensioned to allow a defined gas flow with defined pressure.

Additionally, the system comprises means for measuring a sulphur dioxide/carbon dioxide ratio of the exhaust gas. Further, the system comprises a control system configured to compare the measured sulphur dioxide/carbon dioxide ratios.

For example, the means for measuring the sulphur dioxide/carbon dioxide ratio may comprise semiconductor based sensors. Further, several sensors may be used to enhance measurement accuracy. Further, the semiconductor based sensors may need to be replaced from time to time. Accordingly, the semiconductor based sensors may be configured to be easily replaceable. Examples of the semiconductor based sensors, along with respective advantages and disadvantages are illustrated in Table 2. Further, the operating parameters of different solid state gas sensors are illustrated in Table 3.

Table 2

| Materials | Advantages | Disadvantages |
|---|---|---|
| $SnO_2$ | High sensitivity, Good stability in reducing atmosphere | Low selectivity, Dependence on air humidity |
| $WO_3$ | Good sensitivity to oxidizing gases, Good thermal stability | Low sensitivity to reducing gases, Dependence on air humidity, Slow recovery process |
| $Ga_2O_3$ | High stability, Possibility to operate at high temperatures | Low selectivity, Average sensitivity |
| $In_2O_3$ | High sensitivity to oxidizing gases, Fast response and recovery, Low sensitivity to air humidity | Low stability at low oxygen partial pressure |
| CTO ($CrTiO_x$) | High stability, Low sensitivity to air humidity | Average sensitivity |

Table 3

| Metal oxides | Detecting Gases | Operating temperature | Stability | Compatibility with IC fabrication |
|---|---|---|---|---|
| $SnO_2$ | Reducing gases (CO, $H_2$, $CH_4$, etc.) | 200-400 °C | Excellent | Imperfect |
| $WO_3$ | $NO_x$, $O_3$, $H_2S$, $SO_2$ | 300-500 °C | Excellent | Low |
| $Ga_2O_3$ | $O_2$, CO | 600-900 °C | High | Good |
| $In_2O_3$ | $O_2$, $NO_x$ | 200-400 °C | Moderate | Good |
| $MoO_3$ | $NH_3$, $NO_2$ | 200-450 °C | Moderate | Moderate |
| $TiO_2$ | $O_2$, CO, $SO_2$ | 350-800 °C | Enhanced | Moderate |
| ZnO | $CH_4$, $C_4H_{10}$, $O_3$, $NO_x$ | 250-350 °C | Satisfactory | Good |
| CTO | $H_2S$, $NH_3$, CO, volatile organic compounds | 300-450 °C | High | Imperfect |
| $Fe_2O_3$ | Alcohol, $CH_4$, $NO_2$ | 250-450 °C | Low | Moderate |

In order to measure $SO_2$ emission factor, the carbon fuel content is required. Studies show that the carbon fuel content is $87 \pm 1.5$ % for marine gas oil, marine diesel oil and residual oil. Further, it is assumed that this fraction remains unchanged after fuel burning and that essentially all burnt carbon is emitted as $CO_2$. Hence the $SO_2$ emission factor, $EF(SO_2)$, in grams per kilogram fuel using the atomic and molar masses for C and $SO_2$, respectively, can be calculated by the following equation:

$$EF(SO_2)\left[gkg_{fuel}^{1}\right] = \frac{m(SO_2)}{m(fuel)} = \frac{M(SO_2) \cdot \sum[SO_{2,ppb}]}{M(C)/0.87 \cdot \sum[CO_{2,ppm}]} = 4.6 \frac{\sum[SO_{2,ppb}]}{\sum[CO_{2,ppm}]}$$

Further, assuming that essentially all sulphur is emitted as $SO_2$, the relative sulphur content of the fuel, SFC, may be determined using the following equation:

$$SFC[\%] = \frac{m(S)}{m(fuel)} = \frac{M(S) \cdot \sum[SO_{2,ppb}]}{M(C)/0.87 \cdot \sum[CO_{2,ppm}]} = 0.232 \frac{\sum[SO_{2,ppb}]}{\sum[CO_{2,ppm}]}$$

Further, in an embodiment, the system may comprise a secondary air inlet for an analysis route. Furthermore, the system may comprise a control valve for mixing the exhaust gas received from the gas inlet with air from the secondary air inlet. The control valve is configured to control gas flow to the means for measuring sulphur dioxide/carbon dioxide ratio of the exhaust gas. Further, the outlet of the control valve may feed into a circuit for controlling $CO_2$ amount in the exhaust gas. Accordingly, the circuit may comprise a temperature sensor, a $CO_2$ sensor and a humidity sensor. Finally, an outlet from the circuit may lead to the means for measuring sulphur dioxide/carbon dioxide ratio.

One purpose of mixing air is that some sensors have an operating window for $CO_2$ and $SO_X$ concentration. Accordingly, the exhaust gas may need to be diluted in a controlled manner. In an example, the $CO_2$ concentration may be increased by supplying more ethanol to the heater.

In an embodiment, the burning chamber may further comprise an inlet for a second reference fuel. Accordingly, burning of the second reference fuel may produce a second reference exhaust gas. Further, the means for measuring the sulphur dioxide/carbon dioxide ratio may measure sulphur dioxide/carbon dioxide ratio in the second reference exhaust gas. Additionally, the means for measuring the sulphur dioxide/carbon dioxide ratio may use interpolation to convert the sulphur dioxide/carbon dioxide ratio of the combustion exhaust gas to a sulphur content of the combustion fuel.

In an embodiment, the system may further comprise one or more of means for raising alert, means for removing sulphur from the combustion fuel, means for removing sulphur from the combustion exhaust gas and means for mixing at least two different combustion fuels.

In another embodiment, the system may comprise two or three burning chambers, each comprising an air inlet, means to ignite the fuel and an outlet for exhaust gas from the burning (comprising means for preventing condensation if needed). A first burning chamber would be for a first reference fuel, i.e. comprising an inlet for the first reference fuel. This burning chamber's outlet for exhaust gas may not necessarily need means for preventing condensation, if for example the first reference fuel is always ethanol. A second burning chamber would be for the combustion fuel, i.e. comprising an inlet for the combustion fuel. An optional third burning chamber would be for a second reference fuel, i.e. comprising an inlet for the second reference fuel. The system could also be such that it comprises two burning chambers, one for one or two reference fuels (or more than two reference fuels) and one for the combustion fuel. In all other aspects, the embodiments and means etc. described above apply mutatis mutandis.

In another aspect, an embodiment of the present disclosure provides a method for a continuous measurement of sulphur content of a combustion fuel. Accordingly, the method comprises burning a first amount of a first reference fuel to produce a first reference exhaust gas and measuring a sulphur dioxide/carbon dioxide ratio of the first reference exhaust gas. Further, the method comprises burning a third amount of the combustion fuel to produce a combustion exhaust gas and measuring a sulphur dioxide/carbon dioxide ratio of the combustion exhaust gas. Furthermore, the method comprises comparing the sulphur dioxide/carbon dioxide ratio of the first reference exhaust gas and the sulphur dioxide/carbon dioxide ratio of the combustion exhaust gas. Additionally, the method comprises instructing an actioning system if the sulphur dioxide/carbon dioxide ratio of the combustion exhaust gas exceeds the sulphur dioxide/carbon dioxide ratio of the first reference exhaust gas.

In an embodiment, the actioning system performs one or more actions selected from raising an alert, removing sulphur from the combustion fuel, removing sulphur from the combustion exhaust gas and mixing at least two different combustion fuels.

In an embodiment, the method further comprises burning a second amount of a second reference fuel to produce a second reference exhaust gas. The method may further include measuring a sulphur dioxide/carbon dioxide ratio of the second reference exhaust gas. Additionally, the method may comprise using interpolation to convert the sulphur dioxide/carbon dioxide ratio of the combustion exhaust gas to a sulphur content of the combustion fuel.

In another aspect, an embodiment of the present disclosure provides a method for a continuous measurement of sulphur content of a combustion fuel. The method comprises burning a first amount of a first reference fuel to produce a first reference exhaust gas and measuring a sulphur dioxide/carbon dioxide ratio of the first reference exhaust gas. Further, the method comprises burning a second amount of a second reference fuel to produce a second reference exhaust gas and measuring a sulphur dioxide/carbon dioxide ratio of the second reference exhaust gas. Additionally, the method comprises burning a third amount of the combustion fuel to produce a combustion exhaust gas and measuring a sulphur dioxide/carbon dioxide ratio of the combustion exhaust gas. Further, the method comprises using interpolation to convert the sulphur dioxide/carbon dioxide ratio of the combustion exhaust gas to a sulphur content of the combustion fuel. Furthermore, the method comprises instructing an actioning system if the sulphur content of the combustion fuel exceeds a pre-defined limit.

According to an embodiment, the present disclosure provides a method for checking sulphur content in a petrochemical product, such as for example combustion fuel. The method may be performed in a controlled environment. Typically, the amount of fuels, the amount of air, the amount of moisture in air and the temperature are known and/or controllable. At step 1, a reference fuel with known sulphur content is burnt to get a reference value with a measurement device measuring the exhaust gas. For example, the measurement device may comprise a semiconductor based gas sensor for measuring quantity of sulphur dioxide and quantity of carbon dioxide in the exhaust gas. Further, the measurement device may measure a ratio of the quantity of sulphur dioxide to the quantity of carbon dioxide, or it may calculate this ratio. Subsequently, at step 2, the combustion fuel to be tested is burnt and a measurement value is measured by the measurement device. For example, the semiconductor based gas sensor may be used for measuring the ratio of quantity of sulphur dioxide to quantity of carbon dioxide in the exhaust gas upon burning the combustion fuel. Thereafter, at step 3, if the measurement value is greater than the reference value, an alert is issued. Alternatively, one or more corrective actions may be performed to prevent sulphuric acid pollution.

In another embodiment, the present disclosure provides a method of estimating sulphur content based on interpolation. Accordingly, at step 1, at least two reference fuels having different (and known) sulphur content are burnt (one after another or in parallel burning chambers) to get corresponding reference values using the measurement device. Subsequently, at step 2, the combustion fuel to be tested is burnt to get a measurement value. Thereafter, at step 3, interpolation is used to determine sulphur content in the combustion fuel based on the reference values and the measurement value. Further, at step 4, one or more actions, such as for example mixing of two different fuels from two tanks to achieve cost benefit may be performed based on the sulphur content measured in the combustion fuel. In this method, nonlinearities may be compensated as in any measuring problem and thereafter, linear interpolation is considered to be accurate enough for this purpose.

According to an exemplary embodiment, initially air is supplied into the burning chamber through the air inlet. Subsequently, ethanol is burned in the heater. Further, pipes carrying one or more of the ethanol, the combustion fuel and the exhaust gas are heated if applicable. Subsequently, a waiting is performed until the burning chamber and/or the heater has reached its operating temperature. In some instances, the waiting may be prolonged so that condensed water evaporates and is removed with air flow. Thereafter, the control valve is opened in order to allow a sample of the exhaust gas to be passed on for measuring parameters of the exhaust gas such as temperature, humidity and $CO_2$ in order to determine if the parameters are within respective operating ranges. Accordingly, based on the parameters of the exhaust gas, adjustment of one or more of the temperature of the heater and the amount of air mixed with the sample of the exhaust gas may be performed.

In order to obtain the zero reference, activation of the measurement device is performed to read out zero sulphur reference value. Subsequently, in order to obtain the 1 % reference, the first reference fuel is injected into the burn pot, where the first reference fuel starts burning. Thereafter, a waiting for a predefined time is performed until gas concentrations have stabilized. Subsequently, the 1 % reference sulphur value is measured. Further, the zero reference and 1 % reference may be determined at certain intervals to recalibrate the system.

Subsequently, in order to perform a measurement on a combustion fuel, the combustion fuel is injected into the burning chamber, where the combustion fuel starts burning. Thereafter, a waiting for a predefined time is performed until gas concentrations have stabilized. Further, sulphur value of the combustion fuel is measured using the measurement device. Additionally, measurement of the sulphur content in the combustion fuel may be substantially continuous. Accordingly, whenever the vessel engine is running, the combustion fuel is taken to the burning chamber from the vessel engine's fuel pipes.

Embodiments of the present disclosure enable continuous monitoring of sulphur content in petrochemical products. Such monitoring may be performed in a simple and cost effective manner. Accordingly, expensive instruments such as those for performing X-ray fluorescence analysis, X-ray diffraction, chemical reaction based analysis, gas chromatography etc. may be avoided.

Further, embodiments of the present disclosure enable continuous monitoring of sulphur content at multiple stages of fuel handling. For example, sulphur content in a heavy oil fuel may be monitored while the heavy oil fuel is being loaded into a cargo ship. Similarly, sulphur content of a combustion fuel may be monitored while the fuel is being used in a combustion engine. Accordingly, fuel consumers may be provided with information about sulphur content in petrochemical product in a continuous and real-time basis.

Furthermore, embodiments of the present disclosure enable one or more actions to be performed based on the continuous measurement of sulphur content in petrochemical products. For instance, one or more of the personnel, such as cargo ship operators, may be alerted based on sulphur content in a fuel exceeding a pre-defined limit. Accordingly, corrective action may be performed on a timely basis and adverse effects of burning the fuel with high sulphur content may be avoided.

The corrective action may include, for example, purging the fuel out of the fuel oil system or processing the fuel to reduce sulphur content. Additionally, and/or alternatively, the correction action may comprise one or more means of removing sulphur from the combustion fuel, removing sulphur from the combustion exhaust gas and mixing at least two different combustion fuels. Further, the corrective action may be performed automatically based on the measurement of sulphur content.

EXPERIMENTAL PART

As an example, the parameters associated with the burning chamber may be as follows. The fuel burning speed may be 10 ml / hour, which may accordingly generate roughly 80 W of thermal power. Further, the burn pot may be a cylinder, typically of size 20 mm x 50 mm (diameter x height).

Further, the in flowing air pressure combined and fuel burning speed affects the burning chamber temperature. Preferable values of temperatures in the burning chamber may be: 50 deg. Centigrade at bottom of the burning chamber; 200 deg. Centigrade at top of the burning combustion chamber; 400 deg. Centigrade at beginning of the pipe carrying the exhaust gas. Further, temperature may be measured and $SO_X$ measurement may start when temperature of the burning chamber is high enough. Furthermore, fuel may be injected into the burn chamber continuously or as pulses. Moreover, if the burning chamber needs to be flushed between e.g. different fuel types, a time gap of 10 seconds (depending on chamber volume and air flow) may be provided.

DETAILED DESCRIPTION OF THE DRAWINGS

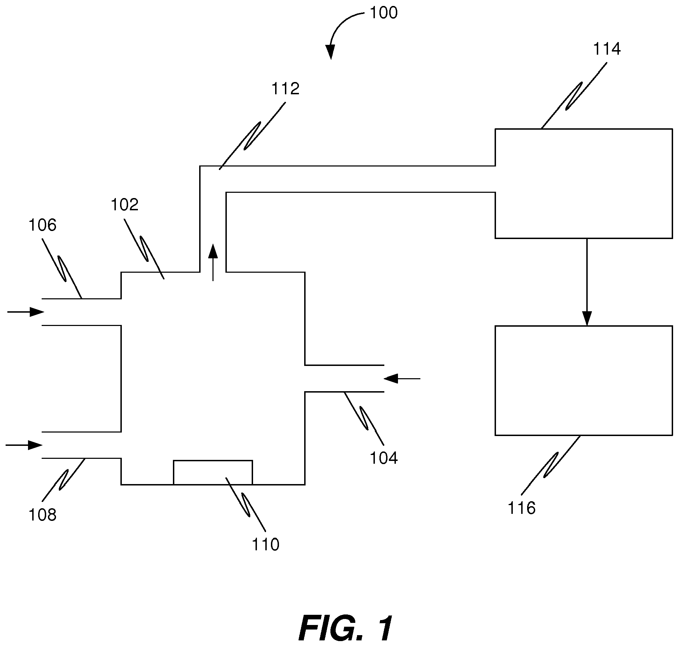

FIG. 1 is a schematic illustration of a system 100 for a continuous measurement of sulphur content of a combustion fuel based on a single reference fuel, according to an embodiment. The system 100 comprises a burning chamber 102. The burning chamber 102 comprises an air inlet 104. Further, the burning chamber 102 comprises an inlet 106 for a first reference fuel. Furthermore, the burning chamber 102 comprises an inlet 108 for the combustion fuel. Additionally, the burning chamber 102 comprises a means 110 to ignite the combustion fuel. Further, the burning chamber 102 comprises an outlet 112 for exhaust gas from the burning. Furthermore, the outlet 112 comprises a means for preventing condensation. Additionally, the system 100 comprises a means 114 for measuring a sulphur dioxide/carbon dioxide ratio of the exhaust gas. Further, the system 100 comprises a control system 116 configured to compare the measured sulphur dioxide/carbon dioxide ratios. Accordingly, sulphur content in the combustion fuel is measured.

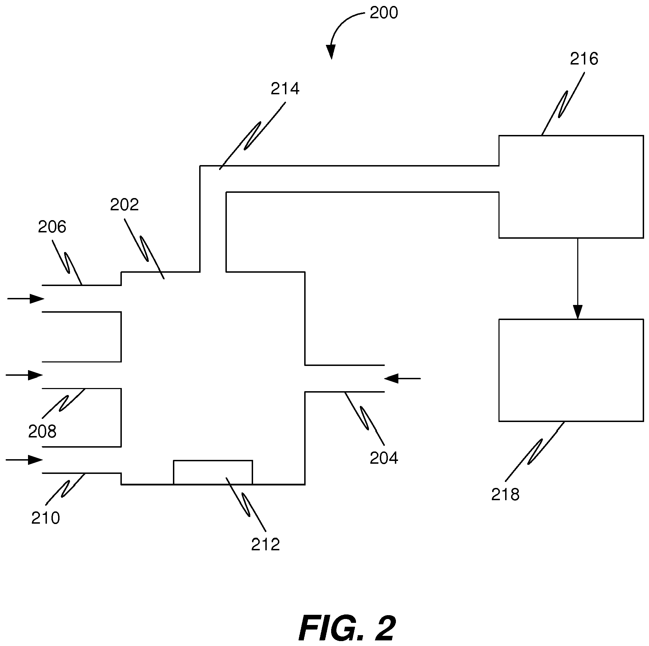

FIG. 2 is a schematic illustration of a system 200 for a continuous measurement of sulphur content of a combustion fuel based on two reference fuels, according to an embodiment. The system 200 comprises a burning chamber 202. The burning chamber 202 comprises an air inlet 204. Further, the burning chamber 202 comprises an inlet 206 for a first reference fuel. Furthermore, the burning chamber 202 comprises an inlet 208 for a second reference fuel. Moreover, the burning chamber 102 comprises an inlet 210 for the combustion fuel. Additionally, the burning chamber 202 comprises a means 212 to ignite the combustion fuel. Further, the burning chamber 202 comprises an outlet 214 for exhaust gas from the burning. Furthermore, the outlet 214 comprises a means for preventing condensation. Additionally, the system 200 comprises a means 216 for measuring a sulphur dioxide/carbon dioxide ratio of the exhaust gas. Furthermore, the system 200 comprises a control system 218 configured to compare the measured sulphur dioxide/carbon dioxide ratios. Further, the control system 218 is configured for using linear interpolation to convert the sulphur dioxide/carbon dioxide ratio of the combustion exhaust gas to a sulphur content of the combustion fuel. Accordingly, sulphur content in the combustion fuel is measured based on burning of two reference fuels with known sulphur content.

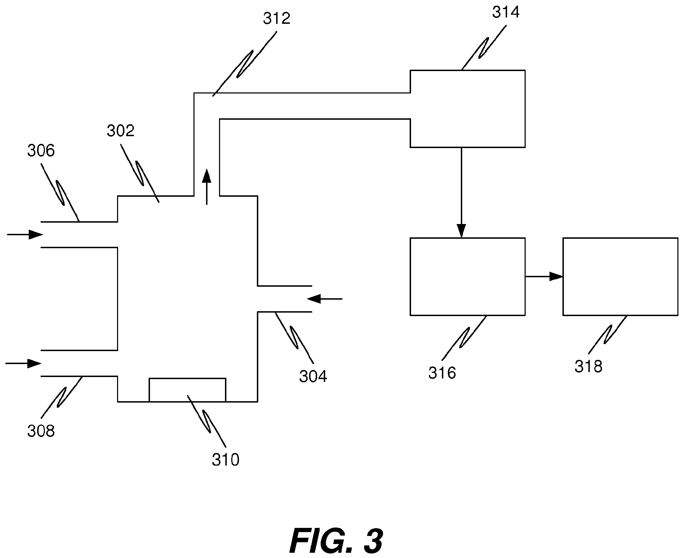

FIG. 3 is a schematic illustration of a system 300 for a continuous measurement of sulphur content of a combustion fuel including an actioning means, according to an embodiment. The system 300 comprises a burning chamber 302. The burning chamber 302 comprises an air inlet 304. Further, the burning chamber 302 comprises an inlet 306 for a first reference fuel. Furthermore, the burning chamber 302 comprises an inlet 308 for the combustion fuel. Additionally, the burning chamber 302 comprises a means 310 to ignite the combustion fuel. Further, the burning chamber 302 comprises an outlet 312 for exhaust gas from the burning. Furthermore, the outlet 312 comprises a means for preventing condensation. Additionally, the system 300 comprises a means 314 for measuring a sulphur dioxide/carbon dioxide ratio of the exhaust gas. Further, the system 300 comprises a control system 316 configured to compare the measured sulphur dioxide/carbon dioxide ratios. Accordingly, sulphur content in the combustion fuel is measured. Further, the system 300 comprises an actioning system 318 configured for performing one or more actions based on the sulphur content thus measured.

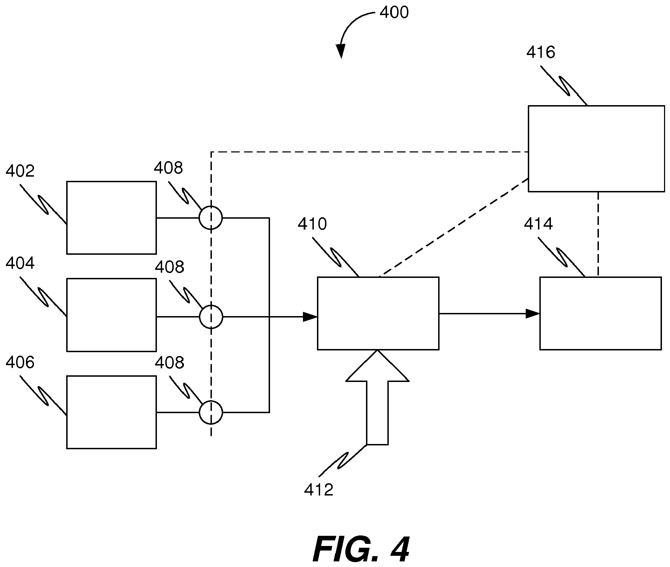

FIG. 4 is a schematic illustration of a system 400 for a continuous measurement of sulphur content of a combustion fuel, according to an embodiment. The system 400 comprises a container 402 for storing a first reference fuel and a container 404 for storing a second reference fuel. Further, the system 400 comprises a container 406 for storing the combustion fuel. Additionally, the system comprises flow control elements 408 configured for controlling supply of corresponding fuel from each of containers 402, 404 and 406 leading to a burning chamber 410. The burning chamber 410 comprises an air inlet 412. Additionally, the burning chamber 410 comprises a means to ignite fuel. Further, the burning chamber 410 comprises an outlet for exhaust gas from the burning. Furthermore, the outlet comprises a means for preventing condensation. Additionally, the system 400 comprises a means, such as for example, a sensor/analyser, for measuring a sulphur dioxide/carbon dioxide ratio of the exhaust gas. Furthermore, the system 400 comprises a control system 416 configured to compare the measured sulphur dioxide/carbon dioxide ratios. Further, the control system 416 is configured for using linear interpolation to convert the sulphur dioxide/carbon dioxide ratio of the combustion exhaust gas to a sulphur content of the combustion fuel. Accordingly, sulphur content in the combustion fuel is measured based on burning of two reference fuels with known sulphur content.

Additionally, the control system 416 is configured to control each of flow control elements 408. Accordingly, the control system 416 can control a quantity of each of the first reference fuel, the second reference fuel and the combustion fuel supplied to the burning chamber. Additionally, the control system 416 can also control a time of supplying each of the first reference fuel, the second reference fuel and the combustion fuel. For instance, the control system may control flow control elements 408 so as to sequentially supply fuels from each of the container 402, container 404 and container 406. Further, the control system 416 may be electrically connected to the burning chamber 410 in order to control the means to ignite the fuel. Accordingly, as an example, subsequent to controlling the flow control elements 408 to supply the first reference fuel, the control system 416 may control the means to ignite the fuel in order to burn the fuels.

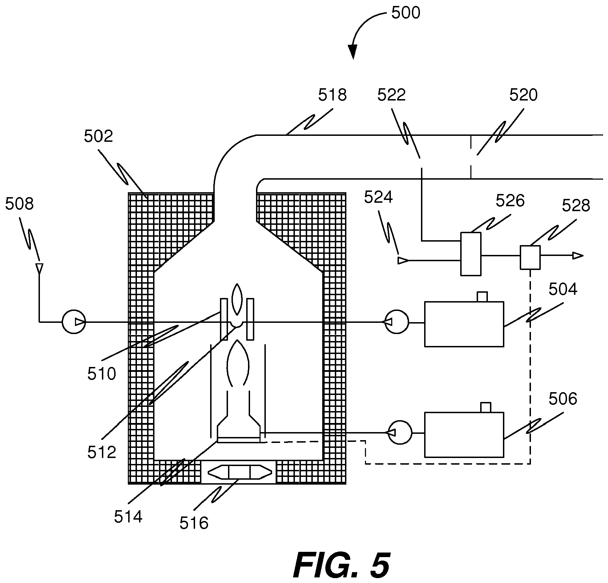

FIG. 5 is a schematic illustration of a system 500 for performing a continuous measurement of sulphur content of a combustion fuel, according to an embodiment. The system 500 comprises a burning chamber 502. Further, the system 500 comprises a container 504 for storing a reference fuel with known sulphur content, such as for example, 1 % sulphur content. Further, an outlet of the container 504 is connected to the burning chamber 502 in order to supply the reference fuel for burning. Additionally, the system 500 comprises a container 506 for storing a burning fuel, such as for example, ethanol (95 %). Accordingly, the container 506 is connected to the burning chamber 502. Further, the system 500 comprises an inlet 508 for receiving a combustion fuel, the sulphur content of which is to be measured. Accordingly, the burning chamber 502 comprises a glow cylinder 510 arranged to be heated by ethanol flame. Alternatively, the glow cylinder 510 may be electrically heated. Further, the burning chamber 502 comprises a burn pot 512 in which each of the reference fuel and the combustion fuel is burnt. Accordingly, the glow cylinder 510 is configured to heat each of the reference fuel and the combustion fuel.

Further, in order to heat the glow cylinder 510, the burning chamber 502 comprises a heater 514. For example, the heater 514 may be a burner configured to burn the burning fuel, such as ethanol, supplied from the container 506. Additionally, the burning chamber 502 comprises an air inlet 516 for providing constant air pressure to the burning chamber 502. For example, an air pump or fan may be used for providing the constant air pressure.

Further, the burning chamber 502 comprises an exhaust gas pipe 518 for carrying the exhaust gas generated from burning of the reference fuel and the combustion fuel. Additionally, the exhaust gas pipe 518 comprises a flow restrictor orifice 520 configured for restricting flow of the exhaust gas in order to facilitate intake of the exhaust gas into gas inlet 522. For example, the flow restrictor orifice 520 may comprise an orifice plate dimensioned to allow a defined gas flow with defined pressure.

Further, the system 500 comprises a secondary air inlet 524 for the analysis route. Furthermore, the system 500 comprises a control valve 526 for mixing the exhaust gas received from the gas inlet 522 with air from the secondary air inlet 524. The control valve 526 is configured to control gas flow to a measurement device (not shown in the figure). Further, the outlet of the control valve 526 feeds into a circuit 528 for controlling $CO_2$ amount in the exhaust gas. Accordingly, the circuit 528 comprises a temperature sensor, a $CO_2$ sensor and a humidity sensor. Finally, an outlet from the circuit 528 leads to the measurement device, which measures the sulphur dioxide/carbon dioxide ratio in the exhaust gas.

Modifications to embodiments of the present disclosure described in the foregoing are possible without departing from the scope of the present disclosure as defined by the accompanying claims. Expressions such as "including", "comprising", "incorporating", "have", "is" used to describe and claim the present disclosure are intended to be construed in a non-exclusive manner, namely allowing for items, components or elements not explicitly described also to be present. Reference to the singular is also to be construed to relate to the plural.

## Claims

1. A system (100, 200, 300, 400, 500) for a continuous measurement of sulphur content of a combustion fuel, comprising

- a burning chamber (102, 202, 302, 410, 502) comprising

  - an air inlet (104, 204, 304, 412),
  - an inlet (106, 206, 306) for a first reference fuel,
  - an inlet (108, 210, 308, 508) for the combustion fuel,
  - means (110, 212, 310) to ignite the fuel,
  - an outlet (112, 214, 312) for exhaust gas from the burning, comprising means for preventing condensation,

- means (114, 216, 314) for measuring a sulphur dioxide/carbon dioxide ratio of the exhaust gas, and
- a control system (116, 218, 316, 416) configured to compare the measured sulphur dioxide/carbon dioxide ratios.

2. A system according to claim 1, wherein the burning chamber further comprises an inlet (208) for a second reference fuel.

3. A system according to claim 1 or 2, wherein the means for preventing condensation are selected from heating means and thermal insulation means.

4. A system according to any of the preceding claims, further comprising at least one of means for raising alert, means for removing sulphur from the combustion fuel, means for removing sulphur from the combustion exhaust gas and means for mixing at least two different combustion fuels.

5. A system according to any of the preceding claims, further comprising an exhaust gas pipe (518) at the outlet for exhaust gas, the exhaust gas pipe comprising a secondary air inlet (524).

6. A system according to claim 5, wherein the exhaust gas pipe further comprises a temperature sensor, a carbon dioxide sensor and a humidity sensor.

7. A system for a continuous measurement of sulphur content of a combustion fuel, comprising

   - a first burning chamber comprising

     - an air inlet,
     - an inlet for a first reference fuel,
     - means to ignite the fuel,
     - an outlet for exhaust gas from the burning, comprising means for preventing condensation,

   - a second burning chamber comprising

     - an air inlet,
     - an inlet for a combustion fuel,
     - means to ignite the fuel,
     - an outlet for exhaust gas from the burning, comprising means for preventing condensation,

   - means for measuring a sulphur dioxide/carbon dioxide ratio of the exhaust gas, and
   - a control system configured to compare the measured sulphur dioxide/carbon dioxide ratios.

8. A system according to claim 7, wherein the first combustion chamber further comprises an inlet for a second reference fuel.

9. A system according to claim 7 or 8, further comprising a third combustion chamber comprising an air inlet, an inlet for a second reference fuel, means to ignite the fuel, and an outlet for exhaust gas from the burning, comprising means for preventing condensation.

10. A vessel comprising a system according to any of the claims 1-9.

11. A vessel according to claim 10, wherein the system is arranged at at least one location selected from a combustion fuel tank inlet, a combustion fuel tank outlet and a combustion engine fuel inlet.

12. A method for a continuous measurement of sulphur content of a combustion fuel, comprising

   - burning a first amount of a first reference fuel to produce a first reference exhaust gas and measuring a sulphur dioxide/carbon dioxide ratio of the first reference exhaust gas,
   - burning a third amount of the combustion fuel to produce a combustion exhaust gas and measuring a sulphur dioxide/carbon dioxide ratio of the combustion exhaust gas,
   - comparing the sulphur dioxide/carbon dioxide ratio of the first reference exhaust gas and the sulphur dioxide/carbon dioxide ratio of the combustion exhaust gas, and
   - instructing an actioning system if the sulphur dioxide/carbon dioxide ratio of the combustion exhaust gas exceeds the sulphur dioxide/carbon dioxide ratio of the first reference exhaust gas.

13. A method according to claim 12, wherein the actioning system performs at least one action selected from raising an alert, removing sulphur from the combustion fuel, removing sulphur from the combustion exhaust gas and mixing at least two different combustion fuels.

14. A method according to claim 12 or 13, further comprising burning a second amount of a second reference fuel to produce a second reference exhaust gas and measuring a sulphur dioxide/carbon dioxide ratio of the second reference exhaust gas, and using linear interpolation to convert the sulphur dioxide/carbon dioxide ratio of the combustion exhaust gas to a sulphur content of the combustion fuel.

15. A method for a continuous measurement of sulphur content of a combustion fuel, comprising

  - burning a first amount of a first reference fuel to produce a first reference exhaust gas and measuring a sulphur dioxide/carbon dioxide ratio of the first reference exhaust gas,
  - burning a second amount of a second reference fuel to produce a second reference exhaust gas and measuring a sulphur dioxide/carbon dioxide ratio of the second reference exhaust gas,
  - burning a third amount of the combustion fuel to produce a combustion exhaust gas and measuring a sulphur dioxide/carbon dioxide ratio of the combustion exhaust gas,
  - using linear interpolation to convert the sulphur dioxide/carbon dioxide ratio of the combustion exhaust gas to a sulphur content of the combustion fuel, and
  - instructing an actioning system if the sulphur content of the combustion fuel exceeds a pre-defined limit.

FIG. 1

**FIG. 2**

**FIG. 3**

FIG. 4

**FIG. 5**

Europäisches
Patentamt

European
Patent Office

Office européen
des brevets

# EUROPEAN SEARCH REPORT

Application Number

EP 17 15 3885

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KR 101 592 022 B1 (TEAM SOLUTION CO LTD [KR]) 7 March 2016 (2016-03-07) * paragraphs [0037] - [0065]; figure 1 * ----- | 1-15 | INV. G01N33/28 |
| X | CN 106 290 804 A (QINGDAO ACAD FOR OPTO-ELECTRONICS ENG) 4 January 2017 (2017-01-04) * figure 2; examples 1, 2 * ----- | 1-15 | |
| A | Anonymous: "RESOLUTION MEPC.184(59)", , 17 July 2009 (2009-07-17), XP055382942, Retrieved from the Internet: URL:http://www.imo.org/en/KnowledgeCentre/ IndexofIMOResolutions/Marine-Environment-P rotection-Committee-(MEPC)/Documents/MEPC. 184(59).pdf [retrieved on 2017-06-20] * pages 20, 21 * ----- | 1-15 | |

TECHNICAL FIELDS
SEARCHED (IPC)

G01N

The present search report has been drawn up for all claims

| Place of search | Date of completion of the search | Examiner |
|---|---|---|
| The Hague | 27 June 2017 | Zwerger, Markus |

CATEGORY OF CITED DOCUMENTS

X : particularly relevant if taken alone
Y : particularly relevant if combined with another
     document of the same category
A : technological background
O : non-written disclosure
P : intermediate document

T : theory or principle underlying the invention
E : earlier patent document, but published on, or
     after the filing date
D : document cited in the application
L : document cited for other reasons

.........................................................................................
& : member of the same patent family, corresponding
     document

EPO FORM 1503 03.82 (P04C01)

**ANNEX TO THE EUROPEAN SEARCH REPORT
ON EUROPEAN PATENT APPLICATION NO.**

EP 17 15 3885

This annex lists the patent family members relating to the patent documents cited in the above-mentioned European search report.
The members are as contained in the European Patent Office EDP file on
The European Patent Office is in no way liable for these particulars which are merely given for the purpose of information.

27-06-2017

| Patent document cited in search report | | Publication date | Patent family member(s) | | Publication date |
|---|---|---|---|---|---|
| KR 101592022 | B1 | 07-03-2016 | KR<br>WO | 101592022 B1<br>2017030314 A1 | 07-03-2016<br>23-02-2017 |
| CN 106290804 | A | 04-01-2017 | NONE | | |

EPO FORM P0459

For more details about this annex : see Official Journal of the European Patent Office, No. 12/82